# EUROPEAN PATENT APPLICATION

(11) **EP 1 394 254 A1**
(43) Date of publication of application: **03.03.2004**
(21) Application number: 02018511.2
(22) Date of filing: 16.08.2002
(51) Int. Cl.: C12N 15/31, C12N 15/10, A61K 39/02

(54) **Method of producing antigens, antigens and their use**

(71) Applicant: Hartung, Thomas, Dr.rer.nat. Dr.med., 78462 Konstanz (DE)
(72) Inventor: Hartung, Thomas, PH Dr.Dr., 78462 Konstanz (DE); Müller, Markus, 78464 Konstanz (DE); Crameri, Reto, Prof. Dr., 7270 Davos Platz (CH)
(74) Representative: TER MEER STEINMEISTER & PARTNER GbR

(57) **Abstract**

A method for producing a peptide having antigenic activity of prokaryotes, eurkaryotes, or viruses which cause persistent diseases in mammals, specifically humans, the peptides produced thereby and their use for diagnosis and for the manufacture of vaccines and/or medicaments.

## Description

The present invention relates to a method for producing a peptide having antigenic activity of prokaryotic or eukaryotic microorganisms and viruses causing persistent diseases in mammals, specifically in humans, the peptides obtained thereby and their use as a diagnostic reagent for the diagnosis of Borreliosis in mammals, specifically in humans, for the manufacture of a vaccine for preventing and a medicament for countering the pathogenic effects caused by Borrelia species, specifically *Borrelia burgdorferi sensu strictu, Borrelia afzelii, Borrelia garinii, Borrelia recurrentis*, *Borrelia burgdorferi sensu lato,* Burgdoferi *andersonii,* Burgdorferi *Japonica, Borrelia hermsii and*/*or Borrelia valaisiana.*

Borreliosis is increasingly recognized as a cause of chronic diseases, such as arthritis, neurological disorders, skin manifestations and arrhythmia. *Borrelia* burgdorferi is known to be the causative agent of Lyme disease, a multi-systemic ailment of humans that is spread through the bite of certain species of *Ixodes* ticks. *Borrelia burgdorferi* is distinguished from other prokaryotics by the extraordinary complexity of its genome, which is composed of a linear chromosome and a variable plasmid complement, consisting of as many as 21 linear and circular plasmids. There is evidence implicating plasmid-encoded proteins in the physiological and antigenic changes which underlie the Lyme disease spirochete's adaptation to different host environments as well as virulence expression within the mammalian host. The near-complete genome of a *Borrelia* burgdorferi B31 clone has been published (C.M. Fraser et al., Nature 390 (1997), 580-586).

While a number of bacterial strains of the species Borrelia are published and available to the public (S. Casjens et al., Journal of Bacteriology (1997), 217-227), the methods for the diagnosis of infections by Borrelia species is difficult and not sufficiently exact and reproducible because the culturing of Borrelia from patient's specimen is difficult because the pathogen is not found in blood, occurs at low numbers in infected tissues and is problematic to grow with doubling times around 20 h, requiring expensive, complex media. Other direct tests for Borrelia like darkfield microscopy or polymerase chain reaction (PCR) lack sensitivity and/or standardization. Therefore, serodiagnosis up to now still represents the method of choice despite several shortcomings, namely that the serodiagnosis of Borreliosis is difficult because of the heterogenity of antigens employed and the lack of standardization. In Europe at least three pathogenic species are encountered, i.e. *Borrelia afzelii (B.a.), Borrelia garinii (B.g.)* and *Borrelia burgdorferi sensu strictu (B.b.s.s.).* The pathogenity of others are being discussed.

The high variability of antigens of each strain further complicates the selection of antigens. It is well established that Borrelia express different surface components in their two hosts, ticks and mammals, respectively, and in culture. The use of antigens of Borrelia culture therefore misses those antigens which are only expressed under pressure of the host's immune system. Furthermore, the batch differences in Borrelia cultures lead to variability of test kit batches, which impair the long-term monitoring of therapeutic effects.

The crude mixture of antigens usually employed for serodiagnosis, i.e. lysates of whole bacteria, results in considerable cross-reactivity of ELISA-assays, e.g. due to anti-flagellin antibodies. The ELISA therefore still requires confirmation by immunoblot, where individual antigens are separated by Western blot, and the presence of respective antibodies can be judged. However, again the variability of the antigen preparations and the lack of standardization for both performance and interpretation make this a highly subjective and labour-intensive test.

The high seroprevalence of Borreliosis (up to 20% in endemic areas), the high rate of infected ticks (up to 40%), the putative link to several chronic manifestations and the poor results of antibiosis in late stages requiring the monitoring of antibody titers, call for novel serological tests based on standardized antigens.

Similar problems arise in the serodiagnosis of other prokaryotic or eukaryotic microorganisms or viruses causing persistent diseases in mammals, such as *Heliobacter pylori,* causing stomach ulcers, *Chlamydia pneumoniae,* considered to cause artherosclerosis, asthma, multiple sclerosis and Alzheimer disease and *Toxoplasma gondii* causing toxoplasmosis, which microorganisms are similarly difficult to diagnose in specimen of mammals and specifically humans suffering from said diseases.

The problem underlying the present invention thus is the provision of a method for producing a peptide having antigenic activity of the above-referred prokaryotic or eukaryotic microorganisms and viruses causing persistent diseases in mammals, specifically in humans, the peptides produced thereby and their use for diagnosis and for the manufacture of vaccines and/or medicaments for preventing and treating the diseases caused by said microorganisms.

It has now been found that the above object can be met by means of a new strategy to identify such antigens, namely by establishing a genomic library of said prokaryotic or eukaryotic microorganisms and cloning the genomic library into phages. By employing a pool of patient sera and the well-established phage/display technique (R. Crameri, Gene Cloning and Analysis: Current Innovations (1997), Horizon Scientific Press, Wymondham, UK, 29-42), those phages carrying relevant epitopes for the library can be selected and the respective proteins identified by sequencing and comparison with the published Borrelia genome (loc. cit.). The proteins were expressed and tested as to their suitability as antigens for serodiagnosis.

The subject-matter of the present invention thus comprises the method for producing a peptide having antigenic activity of prokaryotic or eukaryotic microorganisms and viruses causing persistent diseases in mammals, specifically in humans, as defined in claim 1, the peptides obtained and identified, having antigenic activity of said microorganisms according to claim 12 and the use thereof as a diagnostic reagent for the diagnosis of Borreliosis in mammals, specifically in humans, and for the manufacture of a vaccine for preventing the pathogenic effects or a medicament for countering the pathogenic effects caused by said Borrelia species (claims 14 to 16). The subclaims comprise preferred embodiments of the said subject-matter.

The method of the present invention for producing a peptide having antigenic activity of prokaryotic or eukaryotic microorganisms and viruses causing persistent diseases in mammals, specifically in humans, is characterized by comprising the steps of:
a) cultivating said microorganisms and viruses, isolating and purifying total chromosomal and plasmid DNA thereof and partially digesting said DNA to provide purified DNA fragments;
b) constructing a recombinant phage-display library by ligating said DNA fragments into the DNA of a filamentous bacteriophage and infecting a prokaryotic microorganism with said filamentous bacteriophage;
c) separating and purifying the recombinant phage display library;
d) selecting by affinity the recombinant phages of said library which interact with the sera of patients suffering from said persistent diseases;
e) infecting a prokaryotic microorganism with the selected and purified recombinant phages, sequencing the DNA of the enriched phages by PCR technique and determining the sequences of the separated DNA fragments of the prokaryotic or eukaryotic microorganisms or viruses causing persistent diseases used in step a); and
f) producing and purifying the peptide from said sequences of the DNA fragments of the prokaryotic or eukaryotic microorganisms or viruses causing persistent diseases by means of a prokaryotic microorganism.

The specific biochemical methods used in steps a) to f) of the above method as well as the materials used therefor are standard practice known to the person skilled in the art and will not have to be explained in detail.

In the specific examples of the present invention, the genomic DNA from three Borrelia strains pathogenic for human, i.e. *Borrelia burgdorferi sensu strictu, Borrelia afzelii* and *Borrelia garinii* was cloned into pJuFo and the respective gene products displayed on the filamentous phage using the display technology referred to in the above publications. An independent expression library was constructed for each of the three Borrelia strains.

According to a preferred embodiment of the present invention as prokaryotic or eukaryotic microorganisms causing persistent diseases in mammals to be cultivated in step a) microorganisms of the Borrelia species, specifically *Bor*relia *burgdorferi sensu strictu, Borrelia afzelil, Borrelia garinii, Borrelia recurrentis. Borrelia burgdorferi sensu lato, Borrelia andersonii, Borrelia Japonica, Borrelia herms*ii and/or *Borrelia valaisiana* are being used.

In step a) the DNA of the microorganisms or viruses are digested by means of a restriction enzyme to provide DNA fragments which after purification are inserted via the cutting sections into the vector. The restriction enzymes used preferably are those which are frequent cutters for said DNA and more preferably those which provide sticky ends. Such restriction enzymes are known to the person skilled in the art and available on the market. Preferably, the restriction enzymes *Xba*, *Kpn*I, *Mbo*I and *Bgl*II are being used for cutting the DNA.

According to a preferred embodiment of the present invention, the fragments obtained by partially digesting the DNA in step a) are being purfied by gel-purification and preferably have a size in the range of from 100 bp to 5000 bp, preferably from 356 bp to 2000 bp.

In step b) a filamentous bacteriophage is being used. Preferably use is made of the pJuFo phage surface display technology (R. Crameri et al., Gene 137 (1993), 69-75; R. Crameri et al., Gene 160 (1995), 139), which has been successfully applied to identify allergen repertoires of cDNA libraries from A. *fumigatus* (S. Hemmann et al., Eur. J. Immunol. 28 (1998), 1155-1160; R. Crameri et al., Int. Arch. Allergy Immunol. 110 (1996), 41-45), *Malassezia furfur* (M. Lindborg, J. Invest. Dermatol. 113 (1999), 156-161), *Coprinus* comatus (M. Moser et al., J. Allergy Clin. Immunol. 93 (1994), 1-11), peanuts (T. Kleber-Janke et al., J. Chromatogr. B. Biomed. Sci. Appl. 756 (2001), 295-305; T. Kleber-Janke et al., Int. Arch. Allergy Immunol. 119 (1999), 265-274) and mites (T.L. Eriksson, Eur. J. Biochem. 268 (2001), 287-294) and which allows fast cloning of cDNAs encoding IgE-binding proteins.

According to a preferred embodiment of the present invention, the purified DNA fragments obtained in step a) are inserted into the phagemid vector pJuFo, which is very well suited for the present method and has been described by R. Crameri et al. (R. Crameri et al., Gene 137 (1993), 69-75; R. Crameri et al., Gene 160 (1995), 139). Preferably, the phagemid vector pJuFo is cut with a restriction enzyme providing sticky ends to provide for the correct insertion of the DNA fragements obtained in step a), and more preferably using a compatible or the same restriction enzyme as that used for cutting the DNA of the microorganisms and providing the DNA fragments to be inserted into the phagemid. Most preferably the restriction enzymes *Xba, Kpn*I or *Bgl*II are being used.

Any prokaryotic microorganism useful for the enrichment of the filamentous bacteriophage comprising the inserted DNA fragments can be used, such as *Escherichia coli,* more preferably *Escherichia coli* XL-1-Blue.

According to a preferred embodiment of the present invention the recombinant phage display library obtained in step c) is separated by using the antigens comprised in the pooled sera of patients suffering from said persistent disease by selecting those recombinant phages interacting with said antigens. Preferably, the pooled sera of patients suffering from Borreliosis as the persistent disease is being used.

In step e) of the method of the present invention *Escherichia coli* and preferably the strain XL-1-Blue is being used as the prokaryotic microorganism for enriching the phages.

In the sequencing of DNA of the enriched phages by chain termination reaction (Sanger et al., Proc. Natl. Acad. Sci. USA, 74 (1977), 5463-5467) technique in step e), preferably primers are used selected from the group comprising 5'GGA GTT CAT CCT GGC GGC3', 5'GGC CAG TGA ATT GTA ATA CGA C3' and different length and position variants thereof, which are marked with radioactive groups or at the 5' end, for example by means of fluorescent luminescent or phosphorescent groups, preferably the generally known cyanine dye marker Cy5.

For the identification of the respective Borrelia proteins in step e) the phagemids were analysed by sequencing the inserts. With the obtained DNA-information a BLAST-Search on the NCBI-databases was performed and homology with the known DNA sequence of the genome of the Borrelia species was compared. Those DNA-fragments which showed homology to the Borrelia genome correspond to the DNA sequences SEQ ID NO:1-13 herewith enclosed.

According to a further preferred embodiment of the present invention in step f) the peptides from the selected sequences of the DNA fragments of the prokaryotic or eukaryotic microorganisms causing persistent diseases were prepared by protein expression in a prokaryotic microorganism, preferably as a N-terminal tagged, preferably [His]6-tagged fusion protein in *Escherichia coli* B/21 codon plus cells. Preferably the *Escherichia coli* strain M15 available on the market is being used.

According to a further embodiment, the present invention also relates to peptides having the antigenic activity of the Borrelia species causing persistent diseases, such as Borreliosis in mammals, specifically in humans, characterized by being coded by a nucleotide sequence which presents more than 60% homology, preferably more than 80%, more preferably more than 90%, and specifically more than 95% homology with a nucleotide sequence selected from the group comprising the following DNA-peptide sequences (herewith attached):
SEQ ID NO:1 (bbk32),
SEQ ID NO:2 (bbk32-variant)
SEQ ID NO:3 (ORF-6),
SEQ ID NO:4 (bbp37),
SEQ ID NO:5 (orf27),
SEQ ID NO:6 (bb0371),
SEQ ID NO:7 (bb0355),
SEQ ID NO:8 (bb0713),
SEQ ID NO:9 (flhB),
SEQ ID NO:10 (bb0786),
SEQ ID NO:11 (bb0182),
SEQ ID NO:12 (flgL),
SEQ ID NO:13 (bba26).

A further embodiment of the present invention relates to the use of the peptides coded by the above referred nucleotide sequences as a diagnostic reagent for the diagnosis of Borreliosis in mammals, specifically in humans. This use comprises immobilizing the peptides on an inert matrix, contacting the immobilized peptides with patient's serum and detecting the antibody from the patient's serum specifically bonded to the immobilized peptides using conventional techniques.

Preferably an indirect ELISA-test is being used, comprising adsorbing the peptides to a solid-phase, incubating the immobilized peptides with the patient's serum, washing the solid-phase to remove the non-bound antibody-molecules, incubating the solid-phase with an anti-human IgG-antibody carrying a marker, such as an enzyme, washing the solid-phase, detecting said marker for example by incubating the bound IgG-antibody-molecules with the enzyme substrate and determining the enzyme activity, which is directly proportional to the amount of the specific antibodies present in the patient's serum.

The markers preferably used include enzymes, chromophores, fluorochromes, radioactive molecules or antigenic eptopes. Said markers react with enzyme substrates and are metabolized or altered, resulting in a change of colour, fluorescence or luminescence. Besides classical ELISA a solid phase such as microtiter plates, beads, sticks, combs, chips or other solid materials allowing the detection of antibody binding might be foreseen. Alternatively, the antigen can be marked to allow the detection or quantification of the binding to antibodies of the donor. Besides procedures based on the immobilization of either antigen or donor antibody, procedures based on a change of properties of the antigen/antibody complex might be envisaged, e.g. size and mobility shift.

A further embodiment of the present invention relates to the use of the peptides coded by the above sequences for the manufacture of a vaccine for preventing the pathogenic effects caused by Borrelia species, specifically *Borrelia burgdorferi sensu strictu, Borrelia afzelii, Borrelia garinii, Borrelia recurrentis, Borrelia burgdorferi sensu lato, Borrelia andersonii, Borrelia Japonica, Borrelia hermsii and*/*or Borrelia valaisiana.*

This use comprises the administration of specific amounts of said peptides to the patient sufficient to cause the production of antibodies against this peptide, which antibodies will then help to eliminate any Borrelia species later infecting the patient.

In order to vaccinate the patient, either the mere antigen, combinations of antigens or mixtures of antigens with additives such as adjuvants and immunomodulators can be employed. Furthermore, chemophysical modifications altering the immunological response, e.g. binding on the surface, coupling to immunmodulators, fusion with other antigens and effectors can be envisaged. In addition, the use of the genetical information coding for the respective antigen might be employed for either expressing the antigen in and on transfected cells or microorganisms or by the vaccinated host employing DNA and similar vaccination strategies. The steps to be taken when using the peptides of the present invention are known and available to the skilled person.

According to another embodiment, the present invention also covers the use of the peptides coded by the above nucleotide sequences for the manufacture a medicament for the treatment of pathogenic effects caused by Borrelia species, specifically *Borrelia burgdorferi sensu strictu, Borrelia afzelii, Borrelia garinii, Borrelia recurrentis*, *Borrelia burgdorferi sensu lato, Borrelia andersonii, Borrelia Japonica, Borrelia hermsii and*/*or Borrelia valaisiana.*

This use comprises strategies of therapeutic vaccination, i.e. induction of a healing immune response as well as immunmodulation e.g. by providing tolerance to the respective pathogens. Furthermore, antigen preparations might have therapeutic effects in further diseases e.g. due to their immunmodulatory effects. The antigens do not necessarily have to be applied to the patient, but e.g. immune cells (autologous or heterologous) might be exposed in vitro and then transplanted or protective antibodies, proteins or other molecules reacting to the antigen might be produced and given to the patient.

The invention further relates to the use of the peptides coded by the above nucleotide sequences for the identification of specific immune memory cells by restimulation. This allows to identify persistently infected patients or patient which have undergone the respective infection. The stimulation of immune cells can also be employed for retransplantation after stimulation in oder to improve host defense.

The invention will be explained more in detail based on the following example.

### EXAMPLE

### 1. Cultivation of Borrelia Species and DNA-Purification

The known and freely available strains *Borrelia burgdorferi s.s.* (N40), *Borrelia afzelii* (Vs461) and *Borrelia garinii* (PSTH) used in this example were kindly provided by T. Kamradt (Deutsches Rheumaforschungszentrum, Monbijoustrasse 2A, Berlin,DE). The strains were grown at 33°C in BSK-medium (Sigma-Aldrich-Chemie GmbH, Deisendorf) to a cell density of > 10⁸ cells per milliliter according to the method described by I. Diterich et al. (Infect. Immun. 96 (2001), 687-694). Only strains subcultered less than 10 times were used. Bacteria were harvested by centrifugation (13000 rpm, 20 min) and washed two times in 0,9% NaCl solution. Total chromosomal and plasmid DNA was purified with the QiAmp tissue Kit (Quiagen, Düren, Germany) according to the manufacturer's direction. The DNA was finally eluted with 100 µl Tris/EDTA buffer (pH 8.0). The DNA concentration was determined spectrophotometrically using a GeneQuant II RNA/DNA Calculator (Pharmacia).

### 2. Construction of a Recombinant Phage Display Library

Two micrograms of Borrelia-DNA inserts, obtained by partial digestion with the restriction enzyme *Mbol* (Fermentas, St.-Leon-Rot, DE) were gel-purified (fragments > 356 bp and < 2000 bp).

These inserts were ligated to one microgram of the phagemid pJuFo (loc. cit.) restricted with the commercial restriction enzyme *Bgl*II (Fermentas, St.-Leon-Rot, DE) as follows: The inserts were preheated to 50°C and 1/35 of the whole insert volume was given to the phagemid (eight times every hour on the first day, 27 times every 15 min on the second day of ligation). In addition ligation buffer was added and three times a day one unit of ligase (Fermentas, St. Leon-Rot, DE). The mixture was precipitated after a final 2 hour incubation at 24°C. After electroporation (Gene Pulser^{TM}, Bio-Rad Laboratories, Hercules, CA, USA) of the ligation-mixture into *Escherichia coli* strain XL-1Blue (Stratagene, La Jolla, USA), 5 ml SOC medium (BIO 101, Vista, CA, USA) were added and the culture was shaken at 150 rpm for one hour at 37°C. At this time, 20 ml SB medium (BIO 101, Vista, CA, USA) containing ampicillin (100 µg/ml) (Sigma-Aldrich-Chemie AG, Buchs, CH) and tetracycline (12.5 µg/ml) (Fluka Chemie AG, Buchs, CH) were added and aliquots were plated on agar containing ampicillin to determine the size of the library. The library size for the three Borrelia strains was determined as independent clones on ampicillin agar-plates. The size depended on the efficiency of ligation and electroporation.

The culture was shaken (200 rpm) over night and a 50 ml culture was inoculated at the next day. The culture was grown at 37°C and 300 rpm up to an OD₅₅₀ (optical density at 550 nm) of 0.8. Helper phage VCSM13 (10¹² plaque forming units (pfu), Stratagene, La Jolla, USA) was added at a multiplicity of infection (MOI) of 20. After one hour incubation at 37°C, the culture was shaken for an additional hour at 300 rpm and 37°C. Following that, 70 µg/ml Kanamycin (Sigma-Aldrich-Chemie AG, Buchs, CH) were added and the mixture incubated for 12 hours. The supernatant was cleared by centrifugation (6000 g, 4°C, 20 min). The phages were precipitated with 4% (W/V) PEG 8000 (polyethylene glycol 8000, Sigma) and 3% (W/V) Macl (Sigma) for one hour at 4°C, followed by a centrifugation with 1400 g at 4°C for 30 min. The precipitated phages were solved in TRIS-buffered saline (TBS), pH 7.5 and stored at -20°C.

In this way, the genomic DNA from three human pathogenic Borrelia strains *(Borrelia burgdorferi s.s., Borrelia afzelii and Borrelia garinii)* was cloned and the related gene products displayed on the phagemid pJuFo. An independent expression library was constructed for each of the three Borrelia strains. The size of the library varied between 3.3 x 10⁶ *(B. a.)* and 7.5 x 10⁷ *(B. b. s.s.* and *B. g.).* Independent clones were recovered as ampicillin-resistant colonies. The diversity of the library was checked by restriction enzyme analysis of 12 phagemids with the known commercial restriction enyzme *Pstl* and *Xba.* The inserts showed all different sizes, which is an indication of the high diversity of the library.

### 3. Selection of Antisera

Sera from eight patients with clinical manifestations of Lyme Borreliosis were selected. They showed all high antibody titer in commercial ELISA and the specific bands in Western Blood. These patients include all typical late stage clinical manifestations, as well as newly infected persons.

Since no methodology is available to discriminate the Borrelia species, the patient was infected with, this procedure might not cover all bacterial species. However, recently published distribution of Borrelia in Southern Germany with 53% *B. afzelii,* 18% *B. garinii* and 11% *B. burgdorferi* including 18% double and triple infections in ticks (C. Rauter et al., J. Clin. Microbiol. 40 (2002), 36-43) as well as other multiple infections of the patient, it is likely that the pooled sera include antibodies against all species.

The sera obtained from these patients with standard protocols were pooled and used in the following proceedings.

### 4. Selection of Recombinant Phages Interacting with Patients Sera

To obtain an IgG-coated surface, anti-human-IgG mAB (HRP-Mouse Anti-Human IgG, Zymed Laboratories Inc., San Francisco, USA) were used as capture antibody (cAB) and coated on polystyrene micro titer wells (Greiner, Nürtingen, Germany). Free binding sites were blocked with TBS/3% Milk (TBS=Tris-Buffered-Saline Buffer pH 7.5, milk from Migros, Zürich, CH). 80 µl TBS and 20 µl serum, pooled from the above selected patients, suffering from different stages of Borreliosis, were added to each well. Incubation over night at 4°C followed.

After washing, 10¹¹ phages each of the three phage libraries were pipetted to each well of microtiter plate (Greiner), which has been coated with IgG from the Borrelia patients' sera pool and incubated for 2 hours at 37°C. Adherent phages were eluted by an pH-shift after several washing steps. Enrichment, phage washing, purification and reinfection procedure for further cycles of selection were performed as described elsewhere (R. Crameri et al, Gene 137: (1993), 69-75; R. Crameri et al., Eur. J. Biochem. 226 (1994), 53-58).

Phages were titrated and used to infect *Escherichia coli* XL-1-Blue to prepare new phages for a further cycle of enrichment. The enrichment of phages was monitored by titrating the number of phages in the wash fractions and in the elution fractions.

The number of eluted phages was 5- to 6-fold higher than in the wash-fraction before, indicating an enrichment of phages.

### 5. Sequencing of Enriched Phages

After five rounds of enrichment, the phage population, enriched by ligand/protein interaction, was analyzed. For this purpose, the DNA of 36 phages was extracted, and the size of the inserts was determined by restriction enzyme analysis and agarose gel electrophoresis. Some of the inserts showed the same size in agarose gel. According to the literature it was concluded that clones, showing the same insert, contain the same Borrelia-DNA-fragment.

To identify the respective Borrelia proteins, the phagemids were analyzed by sequencing the inserts.

After five cycles of selection, *Escherichia coli* strain XL-1-Blue were infected with the eluted phages as described above. For identifying the sequence of the enriched phages, a PCR (Polymerase Chain Reaction) was performed on 50 phagemids using the Thermal Sequence fluorescent labelled primer cycle kit (Amersham Pharmacia Biotech, Freiburg). The primer (MWG, Munich, DE) was Cy5 marked at the 5'-end (5'GGA GTT CAT CCT GGC GGC3'). It is of course possible to use other markers instead of the standard marker Cy5. The following sequence gel was performed on a ALFexpress^{TM} gel (Amersham Pharmacia Biotech, Freiburg, DE).

With the obtained DNA-information a BLAST-Search on the National Center for Biotechnology Information databases (NCBI) was performed. Of 50 obtained sequences, 43 showed homology to the Borrelia genome. 5 of them showed homology to the human genome, 2 of them showed no homology with the sequences of the database. From the 43 sequences which encode for a Borrelia protein some were in the wrong orientation, indicating nonsense proteins on the surface of the phages.

The DNA-sequences which encode for a Borrelia protein are summarized in the sequence listings SEQ ID NO:1-13 herewith enclosed.

### 6. Production of the Identified Proteins

Three of the identified proteins, as well as the well-known OspC, were produced as N-terminal fusion proteins, as described by K.A. Brander et al., (J. Allergy Clin. Immunol. 104 (1999), 630-636), such N-terminal [His]₆-tagged fusion proteins, in *Escherichia coli* B/21 codon plus cells. The *E. coli* B/21 codon plus cells are commercially available and used for protein expression. It is of course possible to use other cells for the production of the identified proteins, such as E. coli, yeast, insect cells etc.

In this example, the encoding DNA was amplified by PCR using the primers shown in the following table:

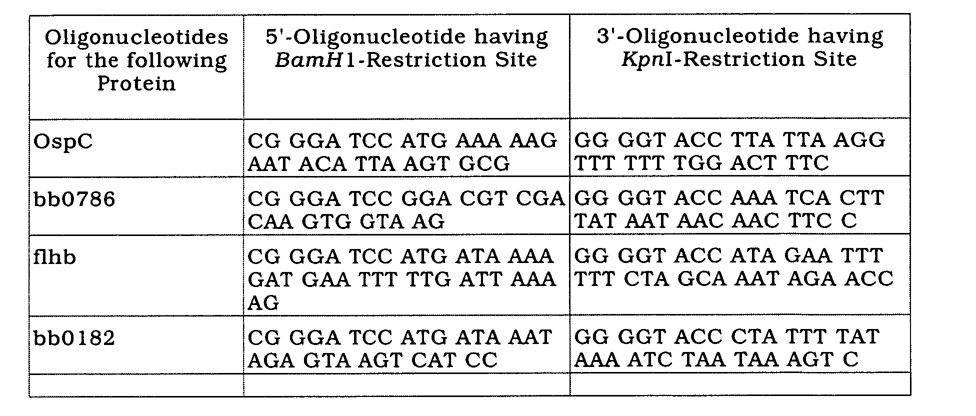

The materials and conditions used in the PCR amplification are available to the person skilled in the art on the market and are used following standard protocols.

The PCR cycling conditions were 94°C for 60s, 56°C for 45s and 72°C for 80s for 30 cycles, followed by a terminal extension cycle at 72°C for 10 min. The amplification products were purified over NucleoSpin columns (Machery-Nagel, Düren, DE), digested with commercial restriction enzymes *BamHI* and KpnI (Fermentas), ligated to *BamH*I/*Kpn*I-restricted commercial restriction vector pQE30 vector (Qiagen) and transformated into *Escherichia coli* strain M15 (Qiagen) by electroporation.

The gene products from positive clones were purified by Ni²⁺-chelate affinity chromatography. The fully denaturated proteins were refolded in vitro by dialysis against TBS/ 1 mM EDTA, pH 7.4 (Pectra/Por® MWCO 3,500, Spectrum® Lab., Houston, US). Determination of protein concentration was performed using the BioRad protein Assay (Bio. Rad., München, DE) with bovine serum albumin as standard. Purity and molecular size of the proteins were analyzed by polyacrylamid gradient gels (4-12%, Invitrogen, Karlsruhe, DE), using standard protocols. The proteins were used for ELISA.

### 7. ELISA

The binding of antibodies of the sera of patients suffering from Borreliosis against the produced peptides was analyzed by a standard direct solid-phase ELISA blocked with TRIS-buffered saline (TBS) (pH 7.5) containing 3% skimmed milk powder (M. Moser et al., J. Allergy Clin. Immunol. 93 (1994), 1-11), using polyclonal mouse anti-human IgG-Antibody (Zymed Laboratories Inc., San Francisco, USA) and the results are expressed as OD. The sera were taken from Borreliosis patients with an age between 15 and 70 years. The optical density (OD) was measured at 405 nm using a reference wavelength of 690 nm. The procedure was performed using standard protocols, such as described in the above publication (M. Moser et al.).

### 8. Expression of Antigens

High level expression vectors carrying the genes from *Borrelia burgdorferi sensu strictu* were used to produce the recombinant proteins in *Escherichia coli.* All constructs were expressed only in small amounts (> 1 mg/1 bacteria culture). After purification of the proteins by affinity-chromatography, they were refolded by dialysis against deionized water.

The purity was checked by SDS-gel-electrophoresis. The dominant band represents calculated size with the OMIGA Software package (Accelrys Ltd., Leeds, UK). There were also some weak bands with a smaller size in the SDS-gel, which represent religated recombinant protein. The band with a size of 38 kDa represents the bacterial Manganese Superoxide Dismutase (MnSOD), which also shows a high affinity to the Ni-NTA-matrix of the chromatography. The Ni-NTA-matrix is a matrix binding proteins which carry His-tag, such as a [His]₆-tag. The product is commercially available from Qiagen. The purity was high enough for testing the proteins in ELISA.

### 9. Evaluation of the Peptides Prepared for Serodiagnosis

Specific IgG to the recombinant proteins obtained was detected in 8 patients with Borreliosis as well as in the sera pool used for the enrichment described above. The sera of all of the patients showed significantly higher ELISA signals than the sera from the control group. The signal intensity of the proteins expressed by the DNA sequences SEQ ID NO:9 (flhB) (0.96 ± 0.13) and SEQ ID NO: 10 (bb0786) (0.47 ± 0.11) is as high as the signal from (OspC) (0.6615 ± 0.20). The protein OspC is a known specific Borrelia protein.

The results of this evaluation are shown in the drawings herewith enclosed, which provide for a comparison of the antibody titer of patient sera versus control sera for the isolated antigens.

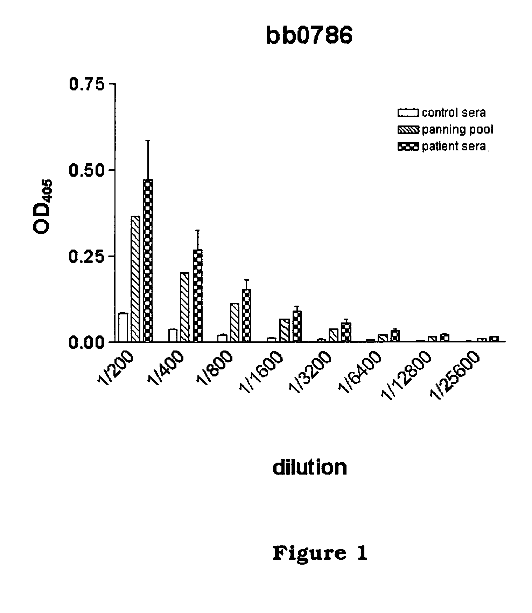

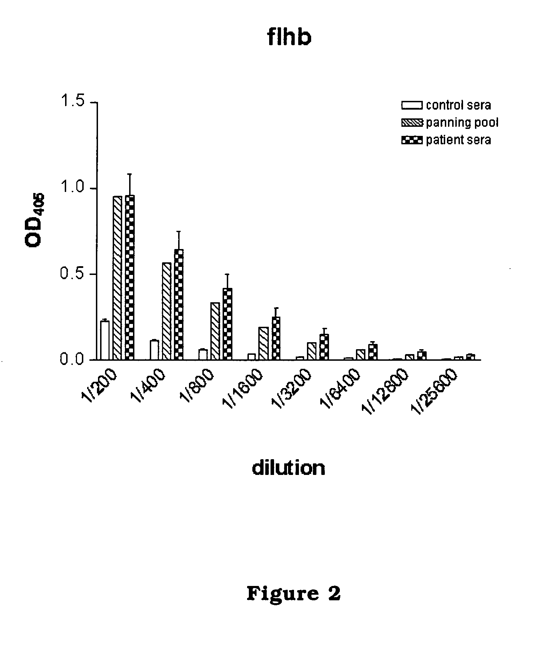

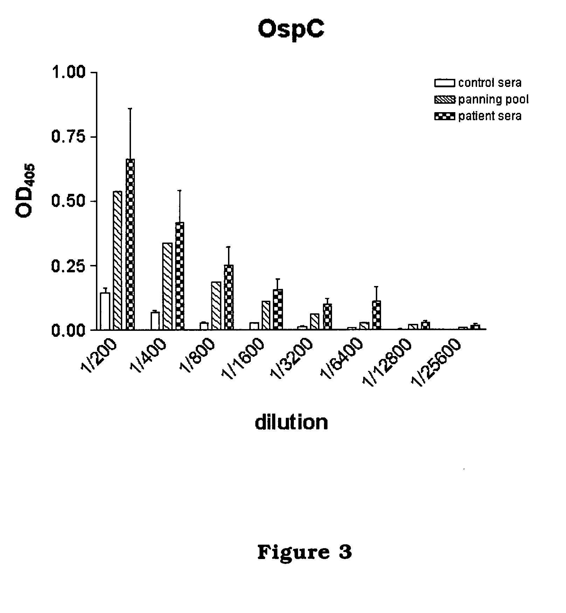

The antigens were immobilized on a 96-well plate and incubated with sera diluted with TBS (pH 7.5) to the dilutions as indicated in the following Figures 1 to 3, the bound IgG using a horseradish peroxidase (HRP)-labelled secondary anti-IgG-antibody. The HRP provides with nitrophenylphosphate (100 µl) a colour reaction, which is being stopped with 2M NaOH (50 µl). The colour reaction of the HRP-enzyme with its substrate is measured as the optical density at 405 nm in an ELISA-reader (Vmax Kinetic Microplate Reader, Molecular Devices, Sunnyvale, CA., USA). The data shown in the Figures 1 to 3 represent the mean value of the signals from the individual sera, the error bars indicating standard deviation. The panning pool is the sera pool, which has been used for the enrichment of the recombinant phages in the above step 3.
**Figure 1** shows the signal intensity obtained with the protein expressed by the DNA sequence SEQ ID NO: 10 (bb0786),
**Figure 2** shows the signal intensity obtained with the protein expressed by the DNA sequence SEQ ID NO:9 (flhB),
**Figure 3** showns the signal intensity of the signal obtained with the protein OspC being a known specific Borrelia protein.

From the drawings it can be seen that the ELISA with affinity purified proteins showed significantly higher signals with patients' sera than with sera from the healthy controls. These data substantiate the fact that the proteins expressed by the DNA sequences herein claimed represent antigenic structures involved in Lyme borreliosis.

## Claims

1. A method for producing a peptide having antigenic activity of prokaryotic or eukaryotic microorganisms or viruses causing persistent diseases in mammals, specifically humans, **characterized by** comprising the steps of:
a) cultivating said microorganisms or viruses, isolating and purifying total chromosomal and plasmid DNA thereof and partially digesting said DNA to provide purified DNA fragments;
b) constructing a recombinant phage-display library by ligating said DNA fragments into the DNA of a filamentous bacteriophage and infecting a prokaryotic microorganism with said filamentous bacteriophage;
c) separating and purifying the recombinant phage display library;
d) selecting by affinity the recombinant phages of said library which interact with the sera of patients suffering from said persistent diseases;
e) infecting a prokaryotic microorganism with the selected and purified recombinant phages, sequencing the DNA of the enriched phages by chain termination reaction technique and determining the sequences of the separated DNA fragments of the prokaryotic or eukaryotic microorganisms or viruses causing persistent diseases used in step a); and
f) producing and purifying the peptide from said sequences of the DNA fragments of the prokaryotic or eukaryotic microorganisms or viruses causing persistent diseases by means of a prokaryotic microorganism.

2. The method according to claim 1, **characterized by** cultivating in step a) microorganisms of the Borrelia species, specifically *Borrelia burgdorferi sensu strictu, Borrelia afzelii. Borrelia garinii, Borrelia recurrentis*, *Borrelia burgdorferi sensu lato, Borrelia andersonii, Borrelia Japonica, Borrelia hermsii* and/or *Borrelia valaisiana.*

3. The method according to claim 1 or 2, **characterized by** partially digesting said DNA using a known restriction enzyme preferably frequent cutter enzymes for said DNA, such as *Xba,* KpnI, MboI or *BglII.*

4. The method according to claim 3, **characterized by** gel-purifying the fragments obtained, preferably having a size in the range of from 100 bp to 5000 bp, preferably from 356 bp to 2000 bp,

5. The method according to claim 1 to 4, **characterized by** in step b) ligating the inserts obtained to the phagemid vector pJuFo restricted using a restriction enzyme, preferably *Xba,* KpnI or *BglII.*

6. The method according to any one of claims 1 to 5, **characterized by** separating the recombinant phage display library using in step d) the antigens comprised in the pooled sera of patients sufffering from said persistant disease by selecting those recombinant phages interacting with said antigens.

7. The method according to claim 6, **characterized by** using in step d) pooled sera of patients suffering from Borreliosis as the persistant disease.

8. The method according to any one of claims 1 to 7, **characterized by** using in step e) as prokaryotic microorganism *Escherichia coli,* preferably of the strain XL-1-Blue.

9. The method according to any one of claims 1 to 8, **characterized by** determining in step e) the sequences the DNA of the enriched phages by PCR using the following primers selected from the group comprising 5'GGA GTT CAT CCT GGC GGC3', 5'GGC CAG TGA ATT GTA ATA CGAC3' and different length and position variants thereof, which are marked with radioactive groups or marked at the 5'-end by means of fluorescent, luminescent or phosphorescent groups, preferably by means of the generally known cyanine dye marker Cy5.

10. The method according to any one of claims 1 to 9, **characterized by** producing in step f) the peptide from said sequences of the DNA fragments by protein expression in a prokaryotic microorganism, preferably as N-terminal [His]₆-tagged fusion protein in *Escherichia coli* B/21 codon plus cells.

11. The method according to claim 1, **characterized by** using in step f) as the prokaryotic microorganisms *Escherichia coli* strain M15.

12. A peptide having antigenic activity of the Borrelia species causing persistant diseases such as Borreliosis in mammals, specifically in humans, **characterized by** being coded by a nucleotide sequence which presents more than 60% homology with a nucleotide sequence selected from the group comprising the following DNA/peptide sequences:
SEQ ID NO:1 (bbk32),
SEQ ID NO:2 (bbk32-variant)
SEQ ID NO:3 (ORF-6),
SEQ ID NO:4 (bbp37),
SEQ ID NO:5 (orf27),
SEQ ID NO:6 (bb0371),
SEQ ID NO:7 (bb0355),
SEQ ID NO:8 (bb0713),
SEQ ID NO:9 (flhB),
SEQ ID NO:10 (bb0786),
SEQ ID NO:11 (bb0182),
SEQ ID NO:12 (flgL),
SEQ ID NO:13 (bba26).

13. The peptide according to claim 12, **characterized in that** that it is being coded by a nucleotide sequence which presents more than 80%, preferably more than 90%, more specifically more than 95% homology with said nucleotide sequences.

14. The use of the peptides according to claims 12 and 13 as a diagnostic reagent for the diagnosis of Borreliosis in mammals, specifically in humans.

15. The use of the peptides according to claims 12 and 13 for the manufacture of a vaccine for preventing the pathogenic effects caused by Borrelia species, specifically *Borrelia burgdorferi sensu strictu, Borrelia afzelii, Borrelia garinii, Borrelia recurrentis*, *Borrelia burgdorferi sensu lato, Borrelia andersonii, Borrelia japonica, Borrelia hermsii* and/or *Borrelia valaisiana.*

16. The use of the peptides according to claims 12 and 13 for the manufacture of a medicament for countering the pathogenic effects caused by Borrelia species, specifically *Borrelia burgdorferi sensu strictu, Borrelia afzelii, Borrelia garinii, Borrelia recurrentis, Borrelia burgdorferi sensu lato, Borrelia andersonii, Borrelia japonica. Borrelia hermsii* and/or *Borrelia valaisiana.*

17. The use of the peptides according to claims 12 and 13 for the identification of specific immune memory cells by restimulation.


SEQUENCE LISTING

<110> Input
Hartung, Thomas
Mueller, Markus
Crameri, Reto

<120> Method for Producing Peptides, Peptides and Their Use

<130> HAR 2003

<160> 13

<170> PatentIn version 3.1

<210> 1
<211> 1065
<212> DNA
<213> Borrelia burgdorferi

<220>
<221> exon
<222> (1)..(1065)
<223> bbk32

<400> 1
atg aaa aaa gtt aaa agt aaa tat ttg gct ttg gga tta ctt ttt ggt 48
Met Lys Lys Val Lys Ser Lys Tyr Leu Ala Leu Gly Leu Leu Phe Gly
1               5                   10                  15

ttt ata agt tgt gat tta ttc ata aga tat gaa atg aaa gag gaa tcc 96
Phe Ile Ser Cys Asp Leu Phe Ile Arg Tyr Glu Met Lys Glu Glu Ser
            20                  25                  30

cct ggc tta ttt gat aag gga aac tct att tta gag act agc gag gaa 144
Pro Gly Leu Phe Asp Lys Gly Asn Ser Ile Leu Glu Thr Ser Glu Glu
        35                  40                  45

tct att aaa aag cct atg aat aag aaa ggt aaa ggt aag att gct aga 192
Ser Ile Lys Lys Pro Met Asn Lys Lys Gly Lys Gly Lys Ile Ala Arg
    50                  55                  60

aag aaa ggc aaa agc aag gtt tct aga aaa gaa ccg tat att cat agt 240
Lys Lys Gly Lys Ser Lys Val Ser Arg Lys Glu Pro Tyr Ile His Ser
65                  70                  75                  80

tta aaa agg gac tct gct aat aaa agc aat ttt tta caa aaa aat gta 288
Leu Lys Arg Asp Ser Ala Asn Lys Ser Asn Phe Leu Gln Lys Asn Val
                85                  90                  95

att tta gag gaa gaa agt tta aaa act gaa tta tta aaa gag caa tct 336
Ile Leu Glu Glu Glu Ser Leu Lys Thr Glu Leu Leu Lys Glu Gln Ser
            100                 105                 110

gag act aga aaa gaa aaa ata caa aaa caa caa gat gaa tat aaa ggg 384
Glu Thr Arg Lys Glu Lys Ile Gln Lys Gln Gln Asp Glu Tyr Lys Gly
        115                 120                 125

atg act caa gga agt tta aat tcc ctt agc ggt gaa agt ggt gaa ttg 432
Met Thr Gln Gly Ser Leu Asn Ser Leu Ser Gly Glu Ser Gly Glu Leu
    130                 135                 140

gag gag cct att gaa agt aat gaa att gat ctt act ata gat tct gat 480
Glu Glu Pro Ile Glu Ser Asn Glu Ile Asp Leu Thr Ile Asp Ser Asp
145                 150                 155                 160

tta agg cca aag agt tcc tta caa ggc att gca gga tca aac tct att 528
Leu Arg Pro Lys Ser Ser Leu Gln Gly Ile Ala Gly Ser Asn Ser Ile
                165                 170                 175

tca tac act gat gaa ata gag gaa gag gat tat gat cag tat tat tta 576
Ser Tyr Thr Asp Glu Ile Glu Glu Glu Asp Tyr Asp Gln Tyr Tyr Leu
            180                 185                 190

gat gaa tat gat gaa gag gat gaa gag gaa ata aga tta agc aat cga 624
Asp Glu Tyr Asp Glu Glu Asp Glu Glu Glu Ile Arg Leu Ser Asn Arg
        195                 200                 205

tat caa tct tat cta gaa ggt gtt aaa tat aat gta gat tca gca att 672
Tyr Gln Ser Tyr Leu Glu Gly Val Lys Tyr Asn Val Asp Ser Ala Ile
    210                 215                 220

caa aca att act aag ata tat aat act tat aca tta ttt tca aca aag 720
Gln Thr Ile Thr Lys Ile Tyr Asn Thr Tyr Thr Leu Phe Ser Thr Lys
225                 230                 235                 240

cta acc caa atg tat tct aca cgc ctt gac aac ttt gct aaa gcc aaa 768
Leu Thr Gln Met Tyr Ser Thr Arg Leu Asp Asn Phe Ala Lys Ala Lys
                245                 250                 255

gct aaa gaa gaa gct gca aag ttt aca aaa gaa gac ctt gaa aaa aat 816
Ala Lys Glu Glu Ala Ala Lys Phe Thr Lys Glu Asp Leu Glu Lys Asn
            260                 265                 270

ttc aag acc tta tta aat tat att caa gta agt gta aag act gca gca 864
Phe Lys Thr Leu Leu Asn Tyr Ile Gln Val Ser Val Lys Thr Ala Ala
        275                 280                 285

aat ttt gta tac ata aat gac acg cat gca aaa agg aaa tta gag aac 912
Asn Phe Val Tyr Ile Asn Asp Thr His Ala Lys Arg Lys Leu Glu Asn
    290                 295                 300

att gaa gca gaa ata aaa act tta att gca aag atc aaa gaa caa tct 960
Ile Glu Ala Glu Ile Lys Thr Leu Ile Ala Lys Ile Lys Glu Gln Ser
305                 310                 315                 320

aat tta tac gaa gca tat aaa gca ata gta acg tca atc tta tta atg 1008
Asn Leu Tyr Glu Ala Tyr Lys Ala Ile Val Thr Ser Ile Leu Leu Met
                325                 330                 335

agg gat tct ctt aaa gaa gtg caa ggt atc att gac aag aat ggc gtt 1056
Arg Asp Ser Leu Lys Glu Val Gln Gly Ile Ile Asp Lys Asn Gly Val
            340                 345                 350

tgg tac taa 1065
Trp Tyr

<210> 2

<211> 818

<212> DNA

<213> Borrelia burgdorferi

<220>

<221> exon

<222> (3)..(818)

<223> bbk32-variant

<400> 2
ca aac aaa agc aat ttt tta caa aaa aat gta att tta gag gaa gaa 47
Asn Lys Ser Asn Phe Leu Gln Lys Asn Val Ile Leu Glu Glu Glu
1               5                   10                  15

agt tta aaa act gaa tta tta aaa gag caa tct gag act aga aaa gaa 95
Ser Leu Lys Thr Glu Leu Leu Lys Glu Gln Ser Glu Thr Arg Lys Glu
                20                  25                  30

aaa ata caa aaa caa caa gat gaa tat aaa ggg atg act caa gga agt 143
Lys Ile Gln Lys Gln Gln Asp Glu Tyr Lys Gly Met Thr Gln Gly Ser
            35                  40                  45

tta aat tcc ctt agc ggt gaa agt ggt gaa ttg gag gag cct att gaa 191
Leu Asn Ser Leu Ser Gly Glu Ser Gly Glu Leu Glu Glu Pro Ile Glu
        50                  55                  60

agt aat gaa att gat ctt act ata gat tct gat tta agg cca aag agt 239
Ser Asn Glu Ile Asp Leu Thr Ile Asp Ser Asp Leu Arg Pro Lys Ser
    65                  70                  75

ttc tta caa ggc att gca gga tca aac tct att tca tac act gat gaa 287
Phe Leu Gln Gly Ile Ala Gly Ser Asn Ser Ile Ser Tyr Thr Asp Glu
80                  85                  90                  95

ata gag gaa gag gat tat gat cgg tat tat tta gat gaa gat gat gaa 335
Ile Glu Glu Glu Asp Tyr Asp Arg Tyr Tyr Leu Asp Glu Asp Asp Glu
                100                 105                 110

gat gat gaa gag gat gaa gag gaa ata aga tta agc aat cga tat caa 383
Asp Asp Glu Glu Asp Glu Glu Glu Ile Arg Leu Ser Asn Arg Tyr Gln
            115                 120                 125

tct tat cta gaa ggt gtt aaa tat aat gta gat tca gca att caa aca 431
Ser Tyr Leu Glu Gly Val Lys Tyr Asn Val Asp Ser Ala Ile Gln Thr
        130                 135                 140

att act aag ata tat aat act tat aca tta ttt tca aca aag cta acc 479
Ile Thr Lys Ile Tyr Asn Thr Tyr Thr Leu Phe Ser Thr Lys Leu Thr
    145                 150                 155

caa atg tat tct aca cgc ctt gac aac ttt gct aaa gcc aaa gct aaa 527
Gln Met Tyr Ser Thr Arg Leu Asp Asn Phe Ala Lys Ala Lys Ala Lys
160                 165                 170                 175

gaa gaa gct gca aag ttt aca aaa gaa gac ctt gaa aaa aat ttc aag 575
Glu Glu Ala Ala Lys Phe Thr Lys Glu Asp Leu Glu Lys Asn Phe Lys
                180                 185                 190

acc tta tta aat tat att caa gta agt gta aag act gca gca aat ttt 623
Thr Leu Leu Asn Tyr Ile Gln Val Ser Val Lys Thr Ala Ala Asn Phe
            195                 200                 205

gta tac ata aat gac aca cat gca aaa agg aaa tta gag aac att gaa 671
Val Tyr Ile Asn Asp Thr His Ala Lys Arg Lys Leu Glu Asn Ile Glu
        210                 215                 220

aca gaa ata aaa act tta att gca aag atc aaa gaa aaa cct gat tta 719
Thr Glu Ile Lys Thr Leu Ile Ala Lys Ile Lys Glu Lys Pro Asp Leu
    225                 230                 235

tac caa gca tat aaa gca ata gta acg cca atc tta tta atg agg gat 767
Tyr Gln Ala Tyr Lys Ala Ile Val Thr Pro Ile Leu Leu Met Arg Asp
240                 245                 250                 255

tct ctt aaa gaa gtg caa agt gcc att gac aag aat ggc att tgg tac 815
Ser Leu Lys Glu Val Gln Ser Ala Ile Asp Lys Asn Gly Ile Trp Tyr
                260                 265                 270

taa 818

<210> 3

<211> 777

<212> DNA

<213> Borrelia burgdorferi

<220>

<221> exon

<222> (1)..(777)

<223>  ORF-6; by homology to plasmid cp8.e

<400>  3
atg aac act aat aat tat ttt aat tta aat aat ttc aac atg gat ttt      48
Met Asn Thr Asn Asn Tyr Phe Asn Leu Asn Asn Phe Asn Met Asp Phe
1               5                   10                  15

atg ctc aaa cta ttt caa gat tat caa aat gtg cta aat gaa aat aaa      96
Met Leu Lys Leu Phe Gln Asp Tyr Gln Asn Val Leu Asn Glu Asn Lys
            20                  25                  30

att ctt aaa aat tca cta aaa att tct tct aag cct act aaa aaa gct      144
Ile Leu Lys Asn Ser Leu Lys Ile Ser Ser Lys Pro Thr Lys Lys Ala
        35                  40                  45

tca aaa cca act cct aag ttt tat ttg acc cca aaa gct atc aaa ata      192
Ser Lys Pro Thr Pro Lys Phe Tyr Leu Thr Pro Lys Ala Ile Lys Ile
    50                  55                  60

att gaa aaa tgc gtt aaa aca tta aaa aaa att gac cct att tct ggt      240
Ile Glu Lys Cys Val Lys Thr Leu Lys Lys Ile Asp Pro Ile Ser Gly
65                  70                  75                  80

tgg ttt cta cat cta ctc gca ata agt ggg tgt agg ggg gcc gaa att      288
Trp Phe Leu His Leu Leu Ala Ile Ser Gly Cys Arg Gly Ala Glu Ile
                85                  90                  95

caa aaa gta aaa atg caa gat att act cct cta tta aac aaa act gga      336
Gln Lys Val Lys Met Gln Asp Ile Thr Pro Leu Leu Asn Lys Thr Gly
            100                 105                 110

gaa act ttt tac aat ata aaa gtt aat ata gct aaa aaa aga aat gtc      384
Glu Thr Phe Tyr Asn Ile Lys Val Asn Ile Ala Lys Lys Arg Asn Val
        115                 120                 125

act tgt att aga gaa att gtc atc aaa tct gaa gaa ttt gag gct att      432
Thr Cys Ile Arg Glu Ile Val Ile Lys Ser Glu Glu Phe Glu Ala Ile
    130                 135                 140

caa aaa gct cac gaa aat tat ttt aac gaa aaa aat ctt gac tca cgg      480
Gln Lys Ala His Glu Asn Tyr Phe Asn Glu Lys Asn Leu Asp Ser Arg
145                 150                 155                 160

cgt act tat ctt ttc caa aaa acc aaa cat aaa ttt aaa gat aac caa      528
Arg Thr Tyr Leu Phe Gln Lys Thr Lys His Lys Phe Lys Asp Asn Gln
                165                 170                 175

att gat att atc aat att tct aga aaa ttt aaa aat ctt ctc aaa aaa      576
Ile Asp Ile Ile Asn Ile Ser Arg Lys Phe Lys Asn Leu Leu Lys Lys
            180                 185                 190

tca gga ttt cgt gcc aat aaa tct ctc cat tta ttt aga aat ttg ttt      624
Ser Gly Phe Arg Ala Asn Lys Ser Leu His Leu Phe Arg Asn Leu Phe
        195                 200                 205

att tca tat tta aaa tct aat ggt tat aat tct ttc caa att aaa gaa      672
Ile Ser Tyr Leu Lys Ser Asn Gly Tyr Asn Ser Phe Gln Ile Lys Glu
    210                 215                 220

ctt atg aaa tat cat tca act tcc gaa att gat aat atc tac ggc ctc      720
Leu Met Lys Tyr His Ser Thr Ser Glu Ile Asp Asn Ile Tyr Gly Leu

225                 230                 235                 240

tct gct gct aac aaa att cac gct tat aaa tgc atg aaa aat aac ctt        768
Ser Ala Ala Asn Lys Ile His Ala Tyr Lys Cys Met Lys Asn Asn Leu
                245                 250                 255

aaa ctt tag                                                            777
Lys Leu

<210>  4

<211>  777

<212>  DNA

<213>  Borrelia burgdorferi

<220>

<221>  exon

<222>  (1)..(777)

<223>  bbp37

<400>  4
atg aac act aat aat tat ttt aat tta aat aat ttc aac atg gat ttt         48
Met Asn Thr Asn Asn Tyr Phe Asn Leu Asn Asn Phe Asn Met Asp Phe
1               5                   10                  15

atg ctc aaa cta ttt caa gat tat caa aat gtg cta aat gaa aat aaa         96
Met Leu Lys Leu Phe Gln Asp Tyr Gln Asn Val Leu Asn Glu Asn Lys
            20                  25                  30

att ctt aaa aat tca cta aaa att tct tct aag cct act aaa aaa gct        144
Ile Leu Lys Asn Ser Leu Lys Ile Ser Ser Lys Pro Thr Lys Lys Ala
        35                  40                  45

tca aaa cca act cct aag ttt tat ttg acc cca aaa gct atc aaa ata        192
Ser Lys Pro Thr Pro Lys Phe Tyr Leu Thr Pro Lys Ala Ile Lys Ile
    50                  55                  60

att gaa aaa tgc gtt aaa ata tta aaa aaa att gac cct att tct ggt        240
Ile Glu Lys Cys Val Lys Ile Leu Lys Lys Ile Asp Pro Ile Ser Gly
65                  70                  75                  80

tgg ttt cta cat cta ctc gca ata agt ggg tgt agg ggg gcc gaa att        288
Trp Phe Leu His Leu Leu Ala Ile Ser Gly Cys Arg Gly Ala Glu Ile
                85                  90                  95

caa aaa gta aaa atg caa gat att act cct cta tta aac aaa act gga        336
Gln Lys Val Lys Met Gln Asp Ile Thr Pro Leu Leu Asn Lys Thr Gly
            100                 105                 110

gaa act ttt tac aat ata aaa gtt aat ata gct aaa aaa aga aat gtc        384
Glu Thr Phe Tyr Asn Ile Lys Val Asn Ile Ala Lys Lys Arg Asn Val
        115                 120                 125

act tgt att aga gaa att gtc atc aaa tct gaa gaa ttt gag gct att 432
Thr Cys Ile Arg Glu Ile Val Ile Lys Ser Glu Glu Phe Glu Ala Ile
    130                 135                 140

caa aaa gct cac gaa aat tat ttt aac gaa aaa aat ctt gac tca cgg 480
Gln Lys Ala His Glu Asn Tyr Phe Asn Glu Lys Asn Leu Asp Ser Arg
145                 150                 155                 160

cgt act tat ctt ttc caa aaa acc aaa cat aaa ttt aaa gat aac caa 528
Arg Thr Tyr Leu Phe Gln Lys Thr Lys His Lys Phe Lys Asp Asn Gln
                165                 170                 175

att gat att atc aat att tct aga aaa ttt aaa aat ctt ctc aaa aaa 576
Ile Asp Ile Ile Asn Ile Ser Arg Lys Phe Lys Asn Leu Leu Lys Lys
            180                 185                 190

tca gga ttt cgt gcc aat aaa tct ctc cat tta ttt aga aat ttg ttt 624
Ser Gly Phe Arg Ala Asn Lys Ser Leu His Leu Phe Arg Asn Leu Phe
        195                 200                 205

att tca tat tta aaa tct aat ggt tat aat tct ttc caa att aaa gaa 672
Ile Ser Tyr Leu Lys Ser Asn Gly Tyr Asn Ser Phe Gln Ile Lys Glu
    210                 215                 220

ctt atg aaa tat cat tca act tcc gaa att gat aat atc tac ggc ctc 720
Leu Met Lys Tyr His Ser Thr Ser Glu Ile Asp Asn Ile Tyr Gly Leu
225                 230                 235                 240

tct gct gct aat aaa att cac gct tat aaa tgc atg aaa aat aac ctt 768
Ser Ala Ala Asn Lys Ile His Ala Tyr Lys Cys Met Lys Asn Asn Leu
                245                 250                 255

aaa ctt tag 777
Lys Leu

<210> 5

<211> 777

<212> DNA

<213> Borrelia burgdorferi

<220>

<221> exon

<222> (1)..(777)

<223> orf27

<400> 5
atg gaa act aat aat tat ttt aat tta aat aat ttc aat ata gac ttt 48
Met Glu Thr Asn Asn Tyr Phe Asn Leu Asn Asn Phe Asn Ile Asp Phe
1               5                   10                  15

atg ctc aaa ttc ttc caa gac tat caa aat gta tta aat gaa aac aaa     96
Met Leu Lys Phe Phe Gln Asp Tyr Gln Asn Val Leu Asn Glu Asn Lys
            20                  25                  30

att ctt aaa aat tca cta aaa att tct tct aag cct act aaa aaa gct     144
Ile Leu Lys Asn Ser Leu Lys Ile Ser Ser Lys Pro Thr Lys Lys Ala
        35                  40                  45

tca aaa cca act cca aag ttt tat ttg aat caa aaa att atc aaa ata     192
Ser Lys Pro Thr Pro Lys Phe Tyr Leu Asn Gln Lys Ile Ile Lys Ile
    50                  55                  60

att gaa aaa tgc gtt aaa aca tta aaa caa att gac cca att tct ggt     240
Ile Glu Lys Cys Val Lys Thr Leu Lys Gln Ile Asp Pro Ile Ser Gly
65                  70                  75                  80

tgg ttt tta cat cta ctg gca ata agt ggc tgc aga ggt gcc gaa ctg     288
Trp Phe Leu His Leu Leu Ala Ile Ser Gly Cys Arg Gly Ala Glu Leu
                85                  90                  95

caa aaa gta aaa atg caa gat att act ccc ttt tta agc aaa act gga     336
Gln Lys Val Lys Met Gln Asp Ile Thr Pro Phe Leu Ser Lys Thr Gly
            100                 105                 110

gaa act ttt tac aat ata aaa gta aat gta gct aaa aaa aga aat gtc     384
Glu Thr Phe Tyr Asn Ile Lys Val Asn Val Ala Lys Lys Arg Asn Val
        115                 120                 125

act tgc att aga gaa att gtc ata aaa tct gta gaa ttt gat gct att     432
Thr Cys Ile Arg Glu Ile Val Ile Lys Ser Val Glu Phe Asp Ala Ile
    130                 135                 140

caa aaa gct cac gaa aat tac ttt aat gaa aaa aat ctt gac cct cgg     480
Gln Lys Ala His Glu Asn Tyr Phe Asn Glu Lys Asn Leu Asp Pro Arg
145                 150                 155                 160

cgt act tat ctt ttc caa aaa act aaa cat aaa ttt aaa gat aat caa     528
Arg Thr Tyr Leu Phe Gln Lys Thr Lys His Lys Phe Lys Asp Asn Gln
                165                 170                 175

att agc att atc aat att tct aat aaa ttc aaa aat ctt ctc aaa aag     576
Ile Ser Ile Ile Asn Ile Ser Asn Lys Phe Lys Asn Leu Leu Lys Lys
            180                 185                 190

tca gga ttt cgt gct aat aaa tca ctt cat tta ttt aga aat ttg ttt     624
Ser Gly Phe Arg Ala Asn Lys Ser Leu His Leu Phe Arg Asn Leu Phe
        195                 200                 205

att tca tat tta aaa tct aat ggt tat aat tct ttt caa att aaa gaa     672
Ile Ser Tyr Leu Lys Ser Asn Gly Tyr Asn Ser Phe Gln Ile Lys Glu
    210                 215                 220

ctt atg aaa tat cat tca act tcc gaa att gat aat att tat gga ctc     720
Leu Met Lys Tyr His Ser Thr Ser Glu Ile Asp Asn Ile Tyr Gly Leu
225                 230                 235                 240

tct gct gct aat aaa att cac gct tat aaa tgc atg aaa aat aac ctt     768
Ser Ala Ala Asn Lys Ile His Ala Tyr Lys Cys Met Lys Asn Asn Leu
                245                 250                 255

aaa ctt taa                                                         777
Lys Leu

<210> 6

<211> 1338

<212> DNA

<213> Borrelia burgdorferi

<220>

<221> exon

<222> (1)..(1338)

<223> bb0371

<400> 6
atg gtt aga atg gaa gat att att tct ctt gca aaa aga aaa gga ttt 48
Met Val Arg Met Glu Asp Ile Ile Ser Leu Ala Lys Arg Lys Gly Phe
1               5                   10                  15

gta ttt cag tct tca gag gtt tac ggg ggc ctt tca gga gct tgg gat 96
Val Phe Gln Ser Ser Glu Val Tyr Gly Gly Leu Ser Gly Ala Trp Asp
            20                  25                  30

tat ggt cct ttg ggg gtt gag ctt aaa aag aat ata aag aaa gag tgg 144
Tyr Gly Pro Leu Gly Val Glu Leu Lys Lys Asn Ile Lys Lys Glu Trp
        35                  40                  45

tgg aag agc atg gtg tac ttg cat gaa aat att gta ggt tta gat agt 192
Trp Lys Ser Met Val Tyr Leu His Glu Asn Ile Val Gly Leu Asp Ser
    50                  55                  60

gct att ttt atg cgc cct gaa att tgg aga gca tct ggt cat gtt gat 240
Ala Ile Phe Met Arg Pro Glu Ile Trp Arg Ala Ser Gly His Val Asp
65                  70                  75                  80

ggt ttt tcg gat tct atg gtt gat tgc aaa gat tgt aaa agt aga ttt 288
Gly Phe Ser Asp Ser Met Val Asp Cys Lys Asp Cys Lys Ser Arg Phe
                85                  90                  95

aga gct gat ttt att gat ttg tca aaa aat tgt ccg aat tgc aaa gtt 336
Arg Ala Asp Phe Ile Asp Leu Ser Lys Asn Cys Pro Asn Cys Lys Val
            100                 105                 110

gga aat aat ttt acc tcc cca aga agt ttt aat tta atg ttt aag acc 384
Gly Asn Asn Phe Thr Ser Pro Arg Ser Phe Asn Leu Met Phe Lys Thr
        115                 120                 125

cac att gga gta gtg gag gat agt tct agt gaa gtt tat tta agg cct 432
His Ile Gly Val Val Glu Asp Ser Ser Ser Glu Val Tyr Leu Arg Pro
    130                 135                 140

gag aca gca caa gga att ttt gtt aat ttt aga aat gtt ttg gat tct 480
Glu Thr Ala Gln Gly Ile Phe Val Asn Phe Arg Asn Val Leu Asp Ser
145                 150                 155                 160

tca agg ctt aag att cct ttt ggg att gct cag gta ggt aaa gcg ttt 528
Ser Arg Leu Lys Ile Pro Phe Gly Ile Ala Gln Val Gly Lys Ala Phe
                165                 170                 175

aga aat gag ata gtt act aaa aat ttt ata ttt aga act tgt gag ttt 576
Arg Asn Glu Ile Val Thr Lys Asn Phe Ile Phe Arg Thr Cys Glu Phe
            180                 185                 190

gag caa atg gaa atg cag ttt ttt gtt cat ccc aag caa ata gac gag 624
Glu Gln Met Glu Met Gln Phe Phe Val His Pro Lys Gln Ile Asp Glu
        195                 200                 205

tgg ttt tgt tat tgg cag caa aat aga atg aat ttt ttt ata gaa act 672
Trp Phe Cys Tyr Trp Gln Gln Asn Arg Met Asn Phe Phe Ile Glu Thr
    210                 215                 220

ctt aaa att agt ccc gat aga tta aga ttt aag gcg cat gat tca acg 720
Leu Lys Ile Ser Pro Asp Arg Leu Arg Phe Lys Ala His Asp Ser Thr
225                 230                 235                 240

cag ctt gct cat tat gca aaa gct gca ttt gat att gag tat gaa ttt 768
Gln Leu Ala His Tyr Ala Lys Ala Ala Phe Asp Ile Glu Tyr Glu Phe
                245                 250                 255

ccg ttt gga ttt cag gaa gta gaa gga att cac aat aga ggt aat tat 816
Pro Phe Gly Phe Gln Glu Val Glu Gly Ile His Asn Arg Gly Asn Tyr
            260                 265                 270

gat tta act cag cac gct aaa ttt tct aat aag ccc aaa gta ttt gag 864
Asp Leu Thr Gln His Ala Lys Phe Ser Asn Lys Pro Lys Val Phe Glu
        275                 280                 285

tat cat gat ttg ttg aca aaa gag aaa tat gtg cct tat gtt att gag 912
Tyr His Asp Leu Leu Thr Lys Glu Lys Tyr Val Pro Tyr Val Ile Glu
    290                 295                 300

act tct gct ggt ctt aca agg tct gtt tta atg act ctt tgt gat gct 960
Thr Ser Ala Gly Leu Thr Arg Ser Val Leu Met Thr Leu Cys Asp Ala
305                 310                 315                 320

tat tct gag gaa gag ctc tca gat gga gac aag cgt att gtt tta cgc 1008
Tyr Ser Glu Glu Glu Leu Ser Asp Gly Asp Lys Arg Ile Val Leu Arg
                325                 330                 335

tta cat ccc aag ttg gct cct tac aag att gct ata ttt cct ctt gtt 1056
Leu His Pro Lys Leu Ala Pro Tyr Lys Ile Ala Ile Phe Pro Leu Val
            340                 345                 350

aaa aaa gtt gag ctt act gag att gct aga agg att tat atg gag ctt 1104
Lys Lys Val Glu Leu Thr Glu Ile Ala Arg Arg Ile Tyr Met Glu Leu
        355                 360                 365

tgc gat gat ttt cat ata ttt tac gat gat agt gga aca ata ggt aaa 1152
Cys Asp Asp Phe His Ile Phe Tyr Asp Asp Ser Gly Thr Ile Gly Lys
    370                 375                 380

agg tat aga cgt caa gac gaa ata gga act cct tat tgc gta aca ata 1200
Arg Tyr Arg Arg Gln Asp Glu Ile Gly Thr Pro Tyr Cys Val Thr Ile
385                 390                 395                 400

gat tac aat acg att gag gat gag aca gtt act gtt aga gaa aga aat 1248
Asp Tyr Asn Thr Ile Glu Asp Glu Thr Val Thr Val Arg Glu Arg Asn
                405                 410                 415

agc atg act cag aag aga att ttt att aat gat tta tat tca tac att      1296
Ser Met Thr Gln Lys Arg Ile Phe Ile Asn Asp Leu Tyr Ser Tyr Ile
            420                 425                 430

aaa aca gag att tta aat tac aaa gag gat ttt aat aaa tga              1338
Lys Thr Glu Ile Leu Asn Tyr Lys Glu Asp Phe Asn Lys
        435                 440                 445

<210>  7

<211>  972

<212>  DNA

<213>  Borrelia burgdorferi

<220>

<221>  exon

<222>  (1)..(972)

<223>  bb0355

<400>  7
atg agt agt aaa aaa gaa ata att ctt aaa gta gaa aac tta atg caa      48
Met Ser Ser Lys Lys Glu Ile Ile Leu Lys Val Glu Asn Leu Met Gln
1               5                   10                  15

aca ttc aca aca gga gaa gat ttt tta ttt tgg aaa aac aaa caa aaa      96
Thr Phe Thr Thr Gly Glu Asp Phe Leu Phe Trp Lys Asn Lys Gln Lys
            20                  25                  30

gta aat gcg gta aac aat gtt agc ttt gaa gtt gaa aaa aat aaa act      144
Val Asn Ala Val Asn Asn Val Ser Phe Glu Val Glu Lys Asn Lys Thr
        35                  40                  45

tta gga ctc gta gga gaa tct ggt tgc ggc aaa tct act act ctt cgc      192
Leu Gly Leu Val Gly Glu Ser Gly Cys Gly Lys Ser Thr Thr Leu Arg
    50                  55                  60

tca ata atg caa ctt tac acg cca act tct gga aat att tac ttt aac      240
Ser Ile Met Gln Leu Tyr Thr Pro Thr Ser Gly Asn Ile Tyr Phe Asn
65                  70                  75                  80

gga aaa aac ata act aaa ctt tca aaa aaa gag ctc tta aaa aca aaa      288
Gly Lys Asn Ile Thr Lys Leu Ser Lys Lys Glu Leu Leu Lys Thr Lys
                85                  90                  95

aaa gat atg caa atg gta ttc caa gat ccc cat act tcg ctt gac cca      336
Lys Asp Met Gln Met Val Phe Gln Asp Pro His Thr Ser Leu Asp Pro
            100                 105                 110

aga atg aca ata aaa gaa ata ata gca gaa cca cta gaa ata tac aat      384
Arg Met Thr Ile Lys Glu Ile Ile Ala Glu Pro Leu Glu Ile Tyr Asn
        115                 120                 125

gaa aac aaa att ctt cca aaa aca aaa caa gaa ata gaa caa agg gta 432
Glu Asn Lys Ile Leu Pro Lys Thr Lys Gln Glu Ile Glu Gln Arg Val
    130                 135                 140

aac gaa cta aca gat att gtc gga cta cat aaa agt atg tta acc aga 480
Asn Glu Leu Thr Asp Ile Val Gly Leu His Lys Ser Met Leu Thr Arg
145                 150                 155                 160

tat cct cat gaa ttt tca gga gga caa aga caa aga ata gga att gct 528
Tyr Pro His Glu Phe Ser Gly Gly Gln Arg Gln Arg Ile Gly Ile Ala
                165                 170                 175

aga gca cta gct tta aat cct aag ctt ttg ctt tta gac gaa gcc gtt 576
Arg Ala Leu Ala Leu Asn Pro Lys Leu Leu Leu Leu Asp Glu Ala Val
            180                 185                 190

tct gca ctt gat gta tca atc aga gct caa att tta aat ctt cta aaa 624
Ser Ala Leu Asp Val Ser Ile Arg Ala Gln Ile Leu Asn Leu Leu Lys
        195                 200                 205

gct ctg caa aaa gaa ttc aat ttg tct tat tta ttt att tct cac gat 672
Ala Leu Gln Lys Glu Phe Asn Leu Ser Tyr Leu Phe Ile Ser His Asp
    210                 215                 220

ctt gcc gta gta aaa tat atg agt gat aaa att gct gta atg tac ctt 720
Leu Ala Val Val Lys Tyr Met Ser Asp Lys Ile Ala Val Met Tyr Leu
225                 230                 235                 240

gga gtt atc ctg gaa ctt gca cct aga gaa acg cta ttt tca aat cca 768
Gly Val Ile Leu Glu Leu Ala Pro Arg Glu Thr Leu Phe Ser Asn Pro
                245                 250                 255

att cat cca tat act aaa atg cta ata gca tca att cct gaa atc aac 816
Ile His Pro Tyr Thr Lys Met Leu Ile Ala Ser Ile Pro Glu Ile Asn
            260                 265                 270

cct gaa aaa aga aaa aat aaa aat ata aaa cta gac gag caa act cta 864
Pro Glu Lys Arg Lys Asn Lys Asn Ile Lys Leu Asp Glu Gln Thr Leu
        275                 280                 285

gca aac att aga aaa att cat tta tca acc caa gta cat cca aag ctc 912
Ala Asn Ile Arg Lys Ile His Leu Ser Thr Gln Val His Pro Lys Leu
    290                 295                 300

gaa gaa gta gaa aaa gat cac ttt gta tct aaa tac ctt ttt gat gag 960
Glu Glu Val Glu Lys Asp His Phe Val Ser Lys Tyr Leu Phe Asp Glu
305                 310                 315                 320

atg aac aga taa 972
Met Asn Arg

<210> 8

<211> 762

<212> DNA

<213> Borrelia burgdorferi

<220>

<221> exon

<222> (1)..(762)

<223> bb0713


<400> 8
ttg atg gaa aac aat att gat aca tta aaa aaa ctt gaa gtt ata tat 48
Leu Met Glu Asn Asn Ile Asp Thr Leu Lys Lys Leu Glu Val Ile Tyr
1               5                   10                  15

aaa tct aag ttt gag ctt gaa gaa agg cga aaa agt att ccc aag tat 96
Lys Ser Lys Phe Glu Leu Glu Glu Arg Arg Lys Ser Ile Pro Lys Tyr
            20                  25                  30

ttg gag gcg aaa aaa aat cag att gaa gaa ttg tcg aaa gtt ctt gtt 144
Leu Glu Ala Lys Lys Asn Gln Ile Glu Glu Leu Ser Lys Val Leu Val
        35                  40                  45

gat ttg caa caa aag ttt aag gag tat caa aaa gaa gac tct gct tta 192
Asp Leu Gln Gln Lys Phe Lys Glu Tyr Gln Lys Glu Asp Ser Ala Leu
    50                  55                  60

aag tta gat att caa gat att aat tca aga aag agc aag gct gaa gaa 240
Lys Leu Asp Ile Gln Asp Ile Asn Ser Arg Lys Ser Lys Ala Glu Glu
65                  70                  75                  80

aaa att gat agc att aaa acg caa aga gaa tat gag gct ctt gaa aaa 288
Lys Ile Asp Ser Ile Lys Thr Gln Arg Glu Tyr Glu Ala Leu Glu Lys
                85                  90                  95

gaa ctt cag gtt att att gac gat gaa gtt aca att aga aaa aag atg 336
Glu Leu Gln Val Ile Ile Asp Asp Glu Val Thr Ile Arg Lys Lys Met
            100                 105                 110

aca cat gtt aat ggg ctt aaa act aaa ata gaa aag gaa ata tta gat 384
Thr His Val Asn Gly Leu Lys Thr Lys Ile Glu Lys Glu Ile Leu Asp
        115                 120                 125

gtc aac gag aag cac atc aaa gaa gag gaa tgt ttt aga gct gaa agc 432
Val Asn Glu Lys His Ile Lys Glu Glu Glu Cys Phe Arg Ala Glu Ser
    130                 135                 140

agt agt ttt gag ctg gag ctt tta gaa att gaa aag aaa ctt tta gaa 480
Ser Ser Phe Glu Leu Glu Leu Leu Glu Ile Glu Lys Lys Leu Leu Glu
145                 150                 155                 160

ata gag agt gaa gag tta aat tgt gct tct aaa atg aat gaa gat ttt 528
Ile Glu Ser Glu Glu Leu Asn Cys Ala Ser Lys Met Asn Glu Asp Phe
                165                 170                 175

tta ttt aaa ttt caa aga ata ata aga aat aaa tca aat gga gtt gtg 576
Leu Phe Lys Phe Gln Arg Ile Ile Arg Asn Lys Ser Asn Gly Val Val
            180                 185                 190

cct ttg gtt aac aat gtt tgc aaa ggt tgt cat atg ata ctt cct att 624
Pro Leu Val Asn Asn Val Cys Lys Gly Cys His Met Ile Leu Pro Ile
        195                 200                 205

gaa ttt gca aat aaa gta aga cgt gag ccc aac gat att aaa ttt tgt 672
Glu Phe Ala Asn Lys Val Arg Arg Glu Pro Asn Asp Ile Lys Phe Cys
    210                 215                 220

cct tat tgc agc aga ata ctt tat tat cag gat aaa gtt cag ata agc 720
Pro Tyr Cys Ser Arg Ile Leu Tyr Tyr Gln Asp Lys Val Gln Ile Ser
225                 230                 235                 240

gat gaa ata att cct ggt agt tta gca gat ctt gtt gaa taa 762
Asp Glu Ile Ile Pro Gly Ser Leu Ala Asp Leu Val Glu
                245                 250

<210> 9

<211> 1119

<212> DNA

<213> Borrelia burgdorferi

<220>

<221> exon

<222> (1)..(1119)

<223> flhB

<400> 9
atg ata aaa gat gaa ttt ttg att aaa agt tgg tat att ccc ctt gat 48
Met Ile Lys Asp Glu Phe Leu Ile Lys Ser Trp Tyr Ile Pro Leu Asp
1               5                   10                  15

ttt ttt tcc gca gat gat gag gga aga acc gaa tta cct act gac cag 96
Phe Phe Ser Ala Asp Asp Glu Gly Arg Thr Glu Leu Pro Thr Asp Gln
            20                  25                  30

aaa aag caa aaa gca agc gaa gaa ggc cgg gta tta aag tct act gaa 144
Lys Lys Gln Lys Ala Ser Glu Glu Gly Arg Val Leu Lys Ser Thr Glu
        35                  40                  45

att aat act gcc gtt agt ctt ttg ttg tta ttt gca ttg ttt ttt ttc 192
Ile Asn Thr Ala Val Ser Leu Leu Leu Leu Phe Ala Leu Phe Phe Phe
    50                  55                  60

atg ctt tct tat ttt gct tta gat tta ata gct gtt ttt aaa gag cag 240
Met Leu Ser Tyr Phe Ala Leu Asp Leu Ile Ala Val Phe Lys Glu Gln
65                  70                  75                  80

gcc atc aag ctt cct gaa gtt atg cgt atg agt gtt tat act atg ggt 288
Ala Ile Lys Leu Pro Glu Val Met Arg Met Ser Val Tyr Thr Met Gly
                85                  90                  95

ttt gca tat att aga tct atc atg ggg tat gtc gtt ttg ttt ttt ttc 336
Phe Ala Tyr Ile Arg Ser Ile Met Gly Tyr Val Val Leu Phe Phe Phe
            100                 105                 110

gca tct tta gct gtt aat ttt ttt gtt aat att att caa gta ggc ttt 384

Ala Ser Leu Ala Val Asn Phe Phe Val Asn Ile Ile Gln Val Gly Phe
        115                 120                 125

ttt att act ttt aaa tct ttg gag cca agg tgg gat aaa att agt ttt 432
Phe Ile Thr Phe Lys Ser Leu Glu Pro Arg Trp Asp Lys Ile Ser Phe
    130                 135                 140

aat ttt tcc aga tgg gca aaa aat tct ttt ttt tca gca ggg gct ttt 480
Asn Phe Ser Arg Trp Ala Lys Asn Ser Phe Phe Ser Ala Gly Ala Phe
145                 150                 155                 160

ttc aat ttg ttt aaa agt ttg tta aaa gtt gtt ata ata tgc ttg ata 528
Phe Asn Leu Phe Lys Ser Leu Leu Lys Val Val Ile Ile Cys Leu Ile
                165                 170                 175

tat tat ttt att ata gaa aac aat ata ggc aaa att tct aag ctt tcg 576
Tyr Tyr Phe Ile Ile Glu Asn Asn Ile Gly Lys Ile Ser Lys Leu Ser
            180                 185                 190

gag tat aca ctt caa tct gga att tct att gtg tta gtg att gcc tat 624
Glu Tyr Thr Leu Gln Ser Gly Ile Ser Ile Val Leu Val Ile Ala Tyr
        195                 200                 205

aag ata tgt ttt ttt tca gta atg ttt ttg gca att gta ggg gtg ttt 672
Lys Ile Cys Phe Phe Ser Val Met Phe Leu Ala Ile Val Gly Val Phe
    210                 215                 220

gat tat ttg ttt caa aga tct cag tac att gag agt ttg aaa atg aca 720
Asp Tyr Leu Phe Gln Arg Ser Gln Tyr Ile Glu Ser Leu Lys Met Thr
225                 230                 235                 240

aaa gaa gag gta aag cag gaa aga aag gaa atg gaa ggt gat cct tta 768
Lys Glu Glu Val Lys Gln Glu Arg Lys Glu Met Glu Gly Asp Pro Leu
                245                 250                 255

ctt cga tct aga ata aaa gag aga atg agg gtt att tta agt acc aat 816
Leu Arg Ser Arg Ile Lys Glu Arg Met Arg Val Ile Leu Ser Thr Asn
            260                 265                 270

tta aga gta gct att cct caa gca gat gta gta att aca aat cca gaa 864
Leu Arg Val Ala Ile Pro Gln Ala Asp Val Val Ile Thr Asn Pro Glu
        275                 280                 285

cat ttt gca gtt gct att aaa tgg gat agc gaa aca atg tta gct cca 912
His Phe Ala Val Ala Ile Lys Trp Asp Ser Glu Thr Met Leu Ala Pro
    290                 295                 300

aag gtg ctt gca aaa ggt caa gat gaa ata gct ctc aca att aaa aaa 960
Lys Val Leu Ala Lys Gly Gln Asp Glu Ile Ala Leu Thr Ile Lys Lys
305                 310                 315                 320

att gca aga gaa aat aat gtt cct tta atg gaa aat aag ctc ctt gca 1008
Ile Ala Arg Glu Asn Asn Val Pro Leu Met Glu Asn Lys Leu Leu Ala
                325                 330                 335

aga gct ctt tat gct aat gtt aag gtt aat gaa gag att cca aga gaa 1056
Arg Ala Leu Tyr Ala Asn Val Lys Val Asn Glu Glu Ile Pro Arg Glu
            340                 345                 350

tat tgg gag att gtt tca aaa att ctt gtg aga gta tat tct att act 1104
Tyr Trp Glu Ile Val Ser Lys Ile Leu Val Arg Val Tyr Ser Ile Thr
        355                 360                 365

aaa aag ttt aat tag                                                    1119
Lys Lys Phe Asn
    370

<210>  10

<211>  645

<212>  DNA

<213>  Borrelia burgdorferi

<220>

<221>  exon

<222>  (1)..(645)

<223>  bb0786

<400>  10
ttg gga cgt cga caa gtg gta agt caa ctg gtt ttg gtc cag tca ttc        48
Leu Gly Arg Arg Gln Val Val Ser Gln Leu Val Leu Val Gln Ser Phe
1               5                   10                  15

gag ggt tcg aat cct tcc gtc cca gta ttt tac att agg agt ttt ttg        96
Glu Gly Ser Asn Pro Ser Val Pro Val Phe Tyr Ile Arg Ser Phe Leu
            20                  25                  30

gtg gaa aat agc agg gtt tta agt tgc caa tat aga tca agc ttt gga       144
Val Glu Asn Ser Arg Val Leu Ser Cys Gln Tyr Arg Ser Ser Phe Gly
        35                  40                  45

tct tct aat gct cgc aga ata aga gct aag tct gaa ata cca gct gtt       192
Ser Ser Asn Ala Arg Arg Ile Arg Ala Lys Ser Glu Ile Pro Ala Val
    50                  55                  60

gtt tac ggg caa ggt aag gat gtt tca cac ttg aga att aag agt agt       240
Val Tyr Gly Gln Gly Lys Asp Val Ser His Leu Arg Ile Lys Ser Ser
65                  70                  75                  80

gaa ttt aat aag aaa ttt gca aaa ttt acg gat aat act gtc tta ata       288
Glu Phe Asn Lys Lys Phe Ala Lys Phe Thr Asp Asn Thr Val Leu Ile
                85                  90                  95

tta gat gat ggc aag ctt gaa aga tgt gtt ttt gtt aaa gat gct gca       336
Leu Asp Asp Gly Lys Leu Glu Arg Cys Val Phe Val Lys Asp Ala Ala
            100                 105                 110

gaa aat att gcc agc aag ctc att tat cat att gat ttt tac gaa gta       384
Glu Asn Ile Ala Ser Lys Leu Ile Tyr His Ile Asp Phe Tyr Glu Val
        115                 120                 125

gat agg aat gtt gag ctt gaa aaa tat gtt cct att aag ctt att gga       432
Asp Arg Asn Val Glu Leu Glu Lys Tyr Val Pro Ile Lys Leu Ile Gly
    130                 135                 140

gct tct att ggg gtt aaa gaa ggt gga att ttg act gtt tta aaa gag       480

Ala Ser Ile Gly Val Lys Glu Gly Gly Ile Leu Thr Val Leu Lys Glu
145                 150                 155                 160

cag gtt aag gta agg tct ttg ccc ttg gat ttg cca gaa ttc ata gag      528
Gln Val Lys Val Arg Ser Leu Pro Leu Asp Leu Pro Glu Phe Ile Glu
                165                 170                 175

ctt gat tta act cct gtt aat aaa gga gat agc gtt ctt ctt aaa gat      576
Leu Asp Leu Thr Pro Val Asn Lys Gly Asp Ser Val Leu Leu Lys Asp
            180                 185                 190

ctt gtg ttg cct tct aat gtt agg ctt gca gaa aat gac gag aat ttg      624
Leu Val Leu Pro Ser Asn Val Arg Leu Ala Glu Asn Asp Glu Asn Leu
        195                 200                 205

gaa gtt gtt att ata aag tga                                          645
Glu Val Val Ile Ile Lys
    210

<210>  11

<211>  1275

<212>  DNA

<213>  Borrelia burgdorferi


<220>

<221>  exon

<222>  (1)..(1275)

<223>  bb0182


<400>  11
atg ata aat aga gta agt cat cca tta aca tat gaa aat ttc aaa acc       48
Met Ile Asn Arg Val Ser His Pro Leu Thr Tyr Glu Asn Phe Lys Thr
1               5                   10                  15

tct tca gca gag caa gag tct aaa att aca aaa ctt tta gaa aac tta       96
Ser Ser Ala Glu Gln Glu Ser Lys Ile Thr Lys Leu Leu Glu Asn Leu
            20                  25                  30

tac aaa ggc ggc aaa agg att gta aaa tta aga aac gat cca aca ggc      144
Tyr Lys Gly Gly Lys Arg Ile Val Lys Leu Arg Asn Asp Pro Thr Gly
        35                  40                  45

gtt act cac gca ata aga cta gat aac gac ata ttt aag ctt aat gta      192
Val Thr His Ala Ile Arg Leu Asp Asn Asp Ile Phe Lys Leu Asn Val
    50                  55                  60

tac ata aaa aat att gac act tct aaa agc aac ctt aga tat acg gaa      240
Tyr Ile Lys Asn Ile Asp Thr Ser Lys Ser Asn Leu Arg Tyr Thr Glu
65                  70                  75                  80

gga tat tta cag tct ctt aca aat att tta aca cga gct aaa gaa att      288
Gly Tyr Leu Gln Ser Leu Thr Asn Ile Leu Thr Arg Ala Lys Glu Ile

                85                  90                  95

gca att caa gga gca agc gga act tat gaa tca gat gac aaa aaa atg 336
Ala Ile Gln Gly Ala Ser Gly Thr Tyr Glu Ser Asp Asp Lys Lys Met
            100                 105                 110

ata tca aaa gaa gta aat gcc ttg ctt gaa gat gta gtt gca ata gca 384
Ile Ser Lys Glu Val Asn Ala Leu Leu Glu Asp Val Val Ala Ile Ala
        115                 120                 125

aac gcc aaa ggg cct gat gga tac agc ata ttc tcg ggg act aaa att 432
Asn Ala Lys Gly Pro Asp Gly Tyr Ser Ile Phe Ser Gly Thr Lys Ile
    130                 135                 140

gat agc gag gcg ttt aag gta act aga gag aat aaa ata agc aaa aca 480
Asp Ser Glu Ala Phe Lys Val Thr Arg Glu Asn Lys Ile Ser Lys Thr
145                 150                 155                 160

agc aaa gac ggt gcg ggc ccc caa ata atc aaa gta gaa tac aac ggc 528
Ser Lys Asp Gly Ala Gly Pro Gln Ile Ile Lys Val Glu Tyr Asn Gly
                165                 170                 175

aac caa gct gaa aaa aaa aca gag gta tac aat gac att cat atg tca 576
Asn Gln Ala Glu Lys Lys Thr Glu Val Tyr Asn Asp Ile His Met Ser
            180                 185                 190

aat aat tac cct gga aat gta ata ttt ttc tta caa aat caa aat att 624
Asn Asn Tyr Pro Gly Asn Val Ile Phe Phe Leu Gln Asn Gln Asn Ile
        195                 200                 205

atc tca tca ata aac act aat gga ttt gcc gta aaa gaa aat aca aaa 672
Ile Ser Ser Ile Asn Thr Asn Gly Phe Ala Val Lys Glu Asn Thr Lys
    210                 215                 220

att tat att gac aat att gag ata gga cta aca gca gga gat act gct 720
Ile Tyr Ile Asp Asn Ile Glu Ile Gly Leu Thr Ala Gly Asp Thr Ala
225                 230                 235                 240

ctt gac atc gtt gca aaa atc aat gaa tct tca gcg cct gtt gaa gca 768
Leu Asp Ile Val Ala Lys Ile Asn Glu Ser Ser Ala Pro Val Glu Ala
                245                 250                 255

agc att gat ccc gtt tta aac tca ttg tct att aaa act aca act cca 816
Ser Ile Asp Pro Val Leu Asn Ser Leu Ser Ile Lys Thr Thr Thr Pro
            260                 265                 270

cac caa att tgg ata aca gaa gaa aag gaa tca aat gtt tta caa aca 864
His Gln Ile Trp Ile Thr Glu Glu Lys Glu Ser Asn Val Leu Gln Thr
        275                 280                 285

ctt gga ata ctt acc aaa aac aat gat acc aaa cta cct cct tac aac 912
Leu Gly Ile Leu Thr Lys Asn Asn Asp Thr Lys Leu Pro Pro Tyr Asn
    290                 295                 300

ctt tct agt agc aca gaa gtt aga agt agg tct att ttc gat gcg ctt 960
Leu Ser Ser Ser Thr Glu Val Arg Ser Arg Ser Ile Phe Asp Ala Leu
305                 310                 315                 320

att gag ctt aga gac aca ctt tac aat aac aaa gaa gag ctt gtt gga 1008
Ile Glu Leu Arg Asp Thr Leu Tyr Asn Asn Lys Glu Glu Leu Val Gly
                325                 330                 335

agt aga agt ctg gca gaa att gac gaa agc tta aaa aga tta ctt ata 1056

Ser Arg Ser Leu Ala Glu Ile Asp Glu Ser Leu Lys Arg Leu Leu Ile
            340                 345                 350

tcc gtt gca gat ctt gga gca aaa gaa aat aga ctt gat aga agc tat      1104
Ser Val Ala Asp Leu Gly Ala Lys Glu Asn Arg Leu Asp Arg Ser Tyr
        355                 360                 365

gaa aga ata agc aaa gaa gct gca gat atg aaa gaa gat atg att caa      1152
Glu Arg Ile Ser Lys Glu Ala Ala Asp Met Lys Glu Asp Met Ile Gln
    370                 375                 380

tat aca gat ctt gat gta aca aaa gca ata act aat tta aat atg gca      1200
Tyr Thr Asp Leu Asp Val Thr Lys Ala Ile Thr Asn Leu Asn Met Ala
385                 390                 395                 400

agc tta gct tat caa gtg tct ata gga atc tct gct aaa ata atg caa      1248
Ser Leu Ala Tyr Gln Val Ser Ile Gly Ile Ser Ala Lys Ile Met Gln
                405                 410                 415

acg act tta tta gat ttt ata aaa tag                                  1275
Thr Thr Leu Leu Asp Phe Ile Lys
            420

<210>  12

<211>  1278

<212>  DNA

<213>  Borrelia burgdorferi

<220>

<221>  exon

<222>  (1)..(1278)

<223>  flgL

<400>  12
atg ata aat aga gta agt cat cca tta aca tat gaa aat ttc aaa acc        48
Met Ile Asn Arg Val Ser His Pro Leu Thr Tyr Glu Asn Phe Lys Thr
1               5                   10                  15

tct tca gca gag caa gag tct aaa att aca aaa ctt tta gaa aac tta        96
Ser Ser Ala Glu Gln Glu Ser Lys Ile Thr Lys Leu Leu Glu Asn Leu
            20                  25                  30

cac aaa ggc ggc aaa agg att gta aaa tta aga aac gat cca aca ggc       144
His Lys Gly Gly Lys Arg Ile Val Lys Leu Arg Asn Asp Pro Thr Gly
        35                  40                  45

gtt act cac gca ata aga cta gat aac gac ata ttt aag ctt aat gta       192
Val Thr His Ala Ile Arg Leu Asp Asn Asp Ile Phe Lys Leu Asn Val
    50                  55                  60

tac ata aaa ata ttg aca ctt cta aaa gca acc tta gat ata cgg aag       240
Tyr Ile Lys Ile Leu Thr Leu Leu Lys Ala Thr Leu Asp Ile Arg Lys

65                  70                  75                  80

gat att tac agt ctc tta caa ata ttt tta aca cga gct aaa gaa att      288
Asp Ile Tyr Ser Leu Leu Gln Ile Phe Leu Thr Arg Ala Lys Glu Ile
                85                  90                  95

gca att caa gga gca agc gga act tat gaa tca gat gac aaa aaa atg      336
Ala Ile Gln Gly Ala Ser Gly Thr Tyr Glu Ser Asp Asp Lys Lys Met
            100                 105                 110

ata tca aaa gaa gta aat gcc ttg ctt gaa gat gta gtt gca ata gca      384
Ile Ser Lys Glu Val Asn Ala Leu Leu Glu Asp Val Val Ala Ile Ala
        115                 120                 125

aac gcc aaa ggg cct gat gga tac agc ata ttc tcg ggg act aaa att      432
Asn Ala Lys Gly Pro Asp Gly Tyr Ser Ile Phe Ser Gly Thr Lys Ile
    130                 135                 140

gat agc gag gcg ttt aag gta act aga gag aat aaa ata agc aaa aca      480
Asp Ser Glu Ala Phe Lys Val Thr Arg Glu Asn Lys Ile Ser Lys Thr
145                 150                 155                 160

agc aaa gac ggt gcg ggc ccc caa ata atc aaa gta gaa tac aac ggc      528
Ser Lys Asp Gly Ala Gly Pro Gln Ile Ile Lys Val Glu Tyr Asn Gly
                165                 170                 175

aac caa gct gaa aaa aaa aca gag gta tac aat gac att cat atg tca      576
Asn Gln Ala Glu Lys Lys Thr Glu Val Tyr Asn Asp Ile His Met Ser
            180                 185                 190

aat aat tac cct gga aat gta ata ttt ttc tta caa aat caa aat att      624
Asn Asn Tyr Pro Gly Asn Val Ile Phe Phe Leu Gln Asn Gln Asn Ile
        195                 200                 205

atc tca tca ata aac act aat gga ttt gcc gta aaa gaa aat aca aaa      672
Ile Ser Ser Ile Asn Thr Asn Gly Phe Ala Val Lys Glu Asn Thr Lys
    210                 215                 220

att tat att gac aat att gag ata gga cta aca gca gga gat act gct      720
Ile Tyr Ile Asp Asn Ile Glu Ile Gly Leu Thr Ala Gly Asp Thr Ala
225                 230                 235                 240

ctt gac atc gtt gca aaa atc aat gaa tct tca gcg cct gtt gaa gca      768
Leu Asp Ile Val Ala Lys Ile Asn Glu Ser Ser Ala Pro Val Glu Ala
                245                 250                 255

agc att gat ccc gtt tta aac tca tcg tct att aaa act aca act cca      816
Ser Ile Asp Pro Val Leu Asn Ser Ser Ser Ile Lys Thr Thr Thr Pro
            260                 265                 270

cac caa att tgg ata aca gaa gaa aag gaa tca aat gtt tta caa aca      864
His Gln Ile Trp Ile Thr Glu Glu Lys Glu Ser Asn Val Leu Gln Thr
        275                 280                 285

ctt gga ata ctt acc aaa aac aat gat acc aaa cta cct cct tac aac      912
Leu Gly Ile Leu Thr Lys Asn Asn Asp Thr Lys Leu Pro Pro Tyr Asn
    290                 295                 300

ctt tct agt agc aca gaa gtt aga agt agg tcc att ttc gat gcg ctt      960
Leu Ser Ser Ser Thr Glu Val Arg Ser Arg Ser Ile Phe Asp Ala Leu
305                 310                 315                 320

att gag ctt aga gac aca ctt tac aat aac aaa gaa gag ctt gtt gga      1008

Ile Glu Leu Arg Asp Thr Leu Tyr Asn Asn Lys Glu Glu Leu Val Gly
                325                 330                 335

agt aga agt ctg gca gaa att gac gaa agc tta aaa aag att act tat      1056
Ser Arg Ser Leu Ala Glu Ile Asp Glu Ser Leu Lys Lys Ile Thr Tyr
            340                 345                 350

atc cgt tgc aga tct tgg agc aaa aag aaa aat aga ctt gat aga agc      1104
Ile Arg Cys Arg Ser Trp Ser Lys Lys Lys Asn Arg Leu Asp Arg Ser
        355                 360                 365

tat gaa aga ata agc aaa gaa gct gca gat atg aaa gaa gat atg att      1152
Tyr Glu Arg Ile Ser Lys Glu Ala Ala Asp Met Lys Glu Asp Met Ile
    370                 375                 380

caa tat aca gat ctt gat gta aca aaa gca ata act aat tta aat atg      1200
Gln Tyr Thr Asp Leu Asp Val Thr Lys Ala Ile Thr Asn Leu Asn Met
385                 390                 395                 400

gca agc tta gct tat caa gtg tct ata gga atc tct gct aaa ata atg      1248
Ala Ser Leu Ala Tyr Gln Val Ser Ile Gly Ile Ser Ala Lys Ile Met
                405                 410                 415

caa acg act tta tta gat ttt ata aaa tag                              1278
Gln Thr Thr Leu Leu Asp Phe Ile Lys
            420                 425

<210>  13

<211>  132

<212>  DNA

<213>  Borrelia burgdorferi

<220>

<221>  exon

<222>  (1)..(132)

<223>  bba26

<400>  13
gtg aat ttg ttt tat gca aat ata gca gta aca ttg ttt gga att aca       48
Val Asn Leu Phe Tyr Ala Asn Ile Ala Val Thr Leu Phe Gly Ile Thr
1               5                   10                  15

agc ttg cta tgc aat ctg gct tgt gat ttt aag act tgt ttc cag ttt       96
Ser Leu Leu Cys Asn Leu Ala Cys Asp Phe Lys Thr Cys Phe Gln Phe
            20                  25                  30

gcc aaa ata ttt caa att ggt tat ttt gca aat tga                      132
Ala Lys Ile Phe Gln Ile Gly Tyr Phe Ala Asn
        35                  40